Europäisches Patentamt

European Patent Office

Office européen des brevets

(11) Publication number: **0 363 083**
**A2**

# EUROPEAN PATENT APPLICATION

(21) Application number: 89309917.6

(51) Int. Cl.⁵: **A61K 37/02 , C12N 15/24**

(22) Date of filing: 28.09.89

The microorganism(s) has (have) been deposited with Fermentation Research institute JAPAN under number BP - 2088

The applicant has filed a statement in accordance with Rule 28 (4) EPC (issue of a sample only to an expert). Accession number(s) of the deposit(s): FERM BP-2088,Fermentation Research Institute/Japan

(30) Priority: 07.10.88 JP 251989/88
20.01.89 JP 11580/89
30.01.89 JP 17444/89
16.02.89 JP 37056/89
13.04.89 JP 91953/89
23.05.89 JP 129447/89

(43) Date of publication of application:
**11.04.90 Bulletin 90/15**

(84) Designated Contracting States:
**AT BE CH DE ES FR GB GR IT LI NL SE**

(71) Applicant: **Chugai Seiyaku Kabushiki Kaisha**
**5-1, 5-chome, Ukima Kita-ku**
**Tokyo115(JP)**

(72) Inventor: **Abe, Tsukasa**
**617-201, 3-chome Takezono**
**Tsukuba-shi Ibaragi(JP)**
Inventor: **Nagasawa, Toshiro**
**807-302, 2-chome Takezono**
**Tsukuba-shi Ibaragi(JP)**
Inventor: **Matsushita, Jun-Ichi**
**205, Iwata Mansion·1-21-7, Tokura**
**Mishima-shi Shizuoka(JP)**
Inventor: **Neichi, Tomohiro**
**C-205, Cope Nakayama 727-1, Nakayama**
**Gotemba-shi Shizuoka(JP)**
Inventor: **Imai, Nobuo**
**401, Mezon Higashifuji 1390-2, Kawashimada**
**Gotemba-shi Shizuoka(JP)**

(74) Representative: **Davies, Jonathan Mark et al**
**Reddie & Grose 16 Theobalds Road**
**London WC1X 8PL(GB)**

(54) **Pharmaceutical preparation for treatment of thrombocytopenic or platelet function-deficient disease.**

(57) A pharmaceutical preparation containing as an active ingredient a polypeptide having an activity of human B cell stimulating factor, for a treatment of diseases involving thrombocytonpenia or impaired platelet functions. The polypeptide can be a novel polypeptide produced by a TNK-01 cell line established from human liposarcoma, or any other polypeptides having BSF2 activity.

EP 0 363 083 A2

# PHARMACEUTICAL PREPARATION FOR TREATMENT OF THROMBOCYTOPENIC OR PLATELET FUNCTION-DEFICIENT DISEASE

## BACKGROUND OF THE INVENTION

### 1. Field of the Invention

The present invention relates to a pharmaceutical preparations for the treatment of diseases involving thrombocytopenia and/or impaired platelet functions, containing a polypeptide having the activity of B cell stimulating factor-2 (abbreviated as BSF2 hereinafter).

### 2. Description of the Related Art

Megakaryocytes release platelets through the differentiation and maturation by the endomitosis, followed by formation of the demarcation membranes and cleavage of the cytoplasm. The formation of platelets is regulated by thrombopoietin (TPO), a factor which regulates the formation of platelets in response to an increase or a decrease in the number of platelets.

The presence of megakaryocyte colony-stimulating factor (Meg-CSF), which stimulates the division of a megakaryocyte colony-forming unit (CFU-Meg), cells one-step differentiated from multipotent stem cells, and their proliferation and differentiation to megakaryocytes have been reported, but it has not been reported that the Meg-CSF increase platelets by in vivo administration.

So far, although reports have been made suggesting the presence of several factors which regulate platelet formation in plasma of thrombocytopenic animals, plasma and urine of thrombocytopenic patients, a culture supernatant of human embryonic kidney cell line (HEK) or the like, there are no reports confirming the presence of a factor which stimulates the increase of platelets by an in vivo administration thereof. Namely, a fraction purified from plasma of thrombocytopenic rabbits administered with an anti-platelet antibody by ammonium sulfate fractionation, lectin column and high performance liquid chromatography, contaminated with some proteins, significantly increased the uptake of $^{75}$Se into platelets, but did not increase the number of platelets in mice (Hill, R. et al., EXP Hematol. 14: 752, 1986).

Moreover, since a culture supernatant of human embryonic kidney cell line (HEK) increases the uptake of $^{35}$S into platelets, the purification of an active factor was attempted and a protein, thrombopoiesis-stimulating factor (TSF) having a molecular weight of 32,000 and an isoelectric point (pI) of 4.7, was isolated. But, the increase of platelets in blood upon an in vivo administration has not been reported (McDonald, T.P. et al., J. Lab. Clin. Med. 85:59, 1975; McDonald, T.P. et al., Prog. Clin. Biol. Res. 215:215, 1986).

On the other hand, a protein, megakaryocyte stimulatory factor (MSF), having a molecular weight of 15,000 as determined by SDS-PAGE and of 9,000 as determined by gel filtration, and a pI value of 5.1, was purified from a culture supernatant of HEK cells, on the basis of its ability to act on a rat megakaryoblastic leukemia cell line to stimulate the production of platelet factor IV (PF4). MSF was also purified from plasma of thrombocytopenic rabbits. Although the MSF significantly increased PF4 synthesis and the uptake of $^{35}$S-sulfate into platelet proteoglycan, it did not increase platelets by an in vivo administration (Tayrien, G. et al., J. Biol. Chem. 262: 3262, 1987; Greenberg, S.M. et al., ibid. 262, 3269, 1987).

Although Meg-CSF having a molecular weight of 45,000 was purified from the plasma of thrombocytopenic patients (Hoffman, R. et al., J. Clin. Invest. 75: 1174, 1985), it did not have an activity of increasing platelets.

It has been reported that an extract of urine from aplastic anemia patients or idiopathic thrombocytopenic purpura patients increased platelets upon administration to rats. But, the extent of the increase is small, and it has not been purified, and therefore, it is not clear whether (or not) it is TPO (Enomoto, K. et al., Br. J. Haematol. 45: 551, 1980; Kawakita, M. et al., ibid. 48: 609, 1981; Miyake, T. et al., Stem Cell 2: 129, 1982; Ishida, Y. et al., EXP. Hematol. 15: 492, 1987).

As seen from the above, although there are some reports which suggest the presence of factors regulating platelet formation, there are no reports which confirm stimulation by the factors of the increase of platelets upon in vivo administration.

The present inventors, after intensive investigations to find a substance which stimulates the increase of platelets, succeeded in establishing a cell line which produces, on stimulation with interleukin-1 (IL-1), a factor capable of increases of platelets in vivo, from a patient of liposarcoma accompanying an increase of platelets. The present inventors further succeeded in purifying the factor, and found that the factor has the N-terminal amino acid sequence which is the same as that of known BSF2, and that the factor is determined as BSF2 by Western analysis using an antibody to BSF2. Note, it has been known that certain animal cells stimulated with IL-1 produce interleukin-6 (IL-6) (BSF2) from some literatures such as J. Cell., Physiol. 134, 292, 1988, etc.

BSF2 is known as a factor which acts on an activated B cells to induce their differentiation to antibody-producing cells. Recently, a cDNA coding for BSF2 was cloned, and on the basis of a nucleotide sequence of the cDNA and a partial amino acid sequence thereof, the BSF2 was found to be a polypeptide having 184 amino acids represented by the following sequence (I):

Pro Val Pro Pro Gly Glu Asp Ser Lys Asp Val Ala Ala Pro His Arg Gln Pro Leu Thr Ser Ser Glu Arg Ile Asp Lys Gln Ile Arg Tyr Ile Leu Asp Gly Ile Ser Ala Leu Arg Lys Glu Thr Cys Asn Lys Ser Asn Met Cys Glu Ser Ser Lys Glu Ala Leu Ala Glu Asn Asn Leu Asn Leu Pro Lys Met Ala Glu Lys Asp Gly Cys Phe Gln Ser Gly Phe Asn Glu Glu Thr Cys leu Val Lys Ile Ile Thr Gly Leu Leu Glu Phe Glu Val Tyr Leu Glu Tyr Leu Gln Asn Arg Phe Glu Ser Ser Glu Glu Gln Ala Arg Ala Val Gln Met Ser Thr Lys Val Leu Ile Gln Phe Leu Gln Lys Lys Ala Lys Asn Leu Asp Ala Ile Thr Thr Pro Asp Pro Thr Thr Asn Ala Ser Leu Leu Thr Lys Leu Gln Ala Gln Asn Gln Trp Leu Gln Asp Met Thr Thr His Leu Ile Leu Arg Ser Phe Lys Glu Phe Leu Gln Ser Ser Leu Arg Ala Leu Arg Gln Met.

Since this polypeptide has been purified by various researchers, it has several different names, for example, 26 kd protein, myeloma/plasmacytoma growth factor, IFN-β2, hepatocytoma stimulating factor (HSF), macrophage-granulocyte inducing factor, B cell differentiation factor (BCDF), B cell stimulating factor 2 (BSF2) and interleukin-6 (IL-6), etc. In the present invention, this protein is referred to as "BSF2".

As polypeptides having BSF2 activities, native BSF2 produced by human T cells transformed with human T cell leukemia virus (Japanese Unexamined Patent Publication, KOKAI, No. 61-115025); a polypeptide wherein an N-terminal proline of the amino acid sequence (I) is lacking, produced by an osteosarcoma cell line MG-63 induced by IL-1, etc. (Eur. J. Biochem. 168, 543-550, 1987); polypeptides produced by a gene recombination technique described in Japanese Unexamined Patent Publications, KOKAI, Nos. 63-42688, 63-56291 and 63-157996, corresponding to EP-257406; EP-220574; PCTWO 88/00206 or the like, were known.

Reviewing recent reports, it is suggested that the BSF2 induces activated B cells to produce antibodies; stimulates the growth of hybridoma cells, plasmacytoma cells, and myeloma cells; induce the expression of the human leukocyte antigen (HLA) class I; induces hematopoietic stem cells to form colonies; induces hepatocyte to produce acute phase protein; and induces neuroblast to form axon. Nevertheless, the present inventors are not aware of any report which suggests that a polypeptide having BSF2 activities effects an increase of the platelets.


SUMMARY OF THE INVENTION

Accordingly, the present invention provides pharmaceutical preparations containing a polypeptide having BSF2 activity for a treatment of diseases involving thrombocytopenia and/or impaired platelet functions.

The present invention also provides a method of treating diseases involving thrombocytopenia and/or impaired platelet functions, comprising the administration of the above pharmaceutical preparation to a patient.

Moreover, the present invention provides the use of a polypeptide having BSF2 activity for the production of the above pharmaceutical preparation.

The present invention still further provides a TNK-01 cell line (FERM BP-2088) and mutants and derivations thereof capable of producing a polypeptide having BSF2 activity.


BRIEF EXPLANATION OF THE DRAWINGS

Figure 1 is a graph showing TPO activity in a TNK-01 culture supernatant in early passages, measured using mice, wherein -o- represents the number of platelets in blood receiving the supernatant, -△- represents a ratio of AChE positive cells in femur bone marrow cells receiving the supernatant, -●- represents the number of platelets in blood receiving a medium not inoculated with the cells, and -▲- represents a ratio of positive AChE cells in femur bone marrow cells receiving a medium not inoculated with the cells;

Fig. 2 is a graph representing TPO activity in a TNK-01 culture supernatant at early passages (sequential administrations for 7 days), wherein the symbols have the same meanings as described for Fig. 1;

Fig. 3 is a graph comparing the TPO productivity of TNK-01 after long-term passages, in a medium containing human interleukin -1α(IL-1α), and that in a medium not containing human interleukin -1α;

Fig. 4 is a graph showing the TPO activity of long term possessed TNK-01, in a culture supernatant from a medium containing IL-1α, measured in a mouse, wherein -Δ - represents a result for the culture supernatant, -o-represents a result of a medium not inoculated with the cells, and -o- represents a result of a medium containing IL-1α not inoculated with the cells;

Fig. 5 is a graph comparing the number of platelets in a mouse receiving a culture supernatant obtained by culturing long-term passaged TNK-01 cells in a medium containing human IL-1α (-Δ-), and the number of platelets in a mouse receiving a medium not inoculated with the cells (-o-);

Fig. 6 represents an elution profile of high performance gel chromatography using a TSK G3000 SW column, wherein a solid line represents absorbance at 280 nm, and a dotted line represents the number of platelets, and arrows (1) to (5) represent the positions of elution of molecular weight standards;

Fig. 7 represent an elution profile of ion exchange chromatography using DEAE-Sepharose CL-6B;

Fig. 8 represents an elution profile of gel chromatography using TSK-G3000SW;

Fig. 9 represents an elution profile of reverse phase HPLC using Vydac C4 column;

Fig. 10 represents an elution profile of reverse phase HPLC for a further purification of active fraction from Fig. 9;

Fig. 11 represents a result of SDS-PAGE for a TPO preparation purified with the reverse phase HPLC shown in Fig. 10, wherein the detection was carried out using 0.25 coomassie brilliant blue R250;

Fig. 12 represents a result corresponding to that shown in Fig. 11, except that the detection was carried out using an antibody to BSF2;

Fig. 13 is a graph showing the increase of platelets in mice by the present purified TPO.

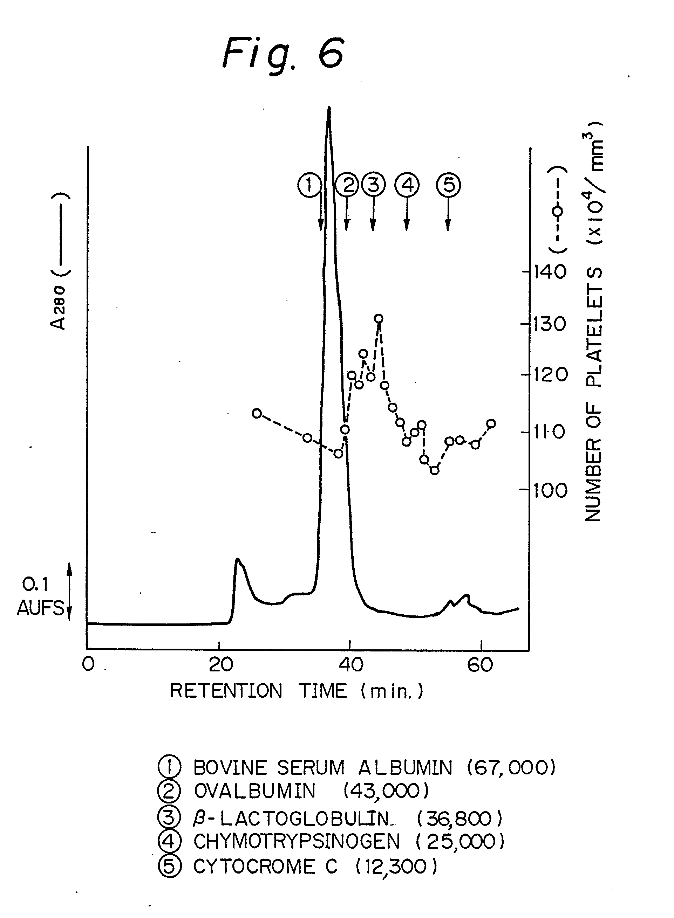

Note, in Figs. 6, 8, 9 and 10, "AUFS" means absorbance unit full scale.

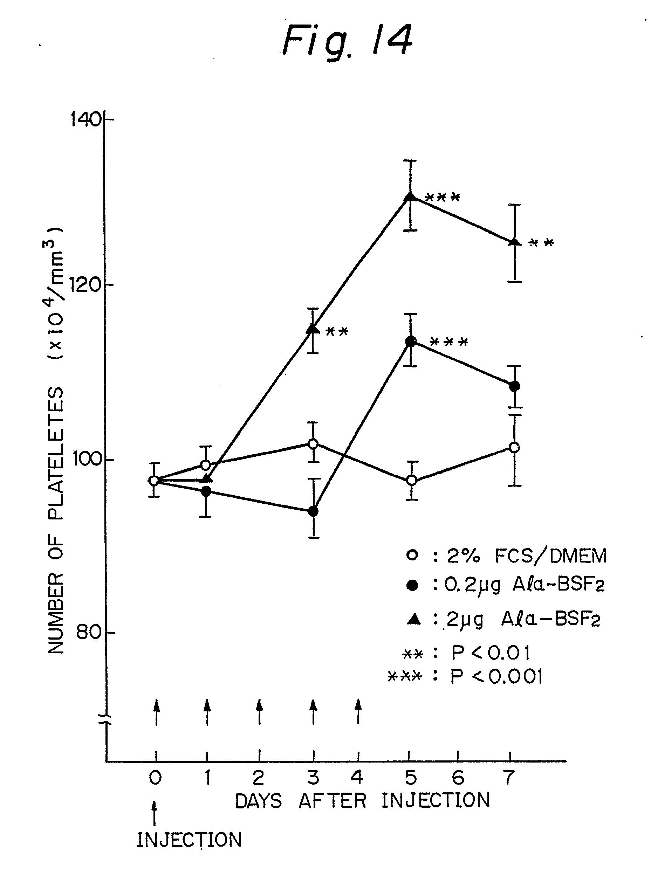

Fig. 14 shows an effect of Ala-BSF2 on the increase of platelets; and

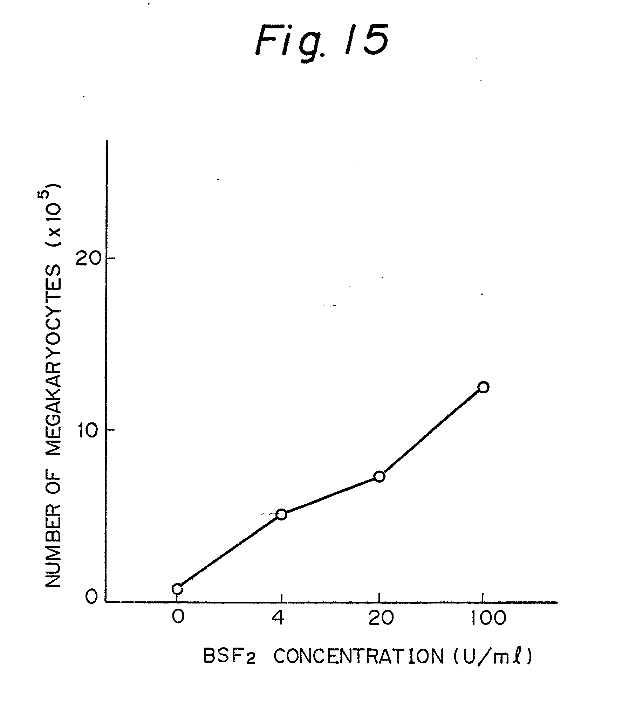

Fig. 15 represents an effect of BSF2 on megakaryocyte colony formation.

## DESCRIPTION OF THE PREFERRED EMBODIMENTS

### Assay method

In the present invention, TPO activity is determined by various methods, described as follows.

### (a) Method using mouse bone marrow cells

Since megakaryocytes and platelets of rodent such as mouse have an acethylcholinesterase (AchE) activity, a differentiation of multipotent stem cells to megakaryocytes can be studied by observing the increase of AChE positive cells by an in vitro bone marrow cell culture. Nevertheless, since this method is not specific to TPO but detects Meg-CSF and erythropoietin, it is used for screening substances including TPO.

Next, as examples of the colony forming method, the soft agar method and plasma clot method, as well as the AChE activity assay method, are described.

### (1) Soft Agar System

Megakaryocyte colony stimulating activity was measured using a soft agar system. 1 to 2 x 10^5 bone marrow cells obtained from male C57B1/6N mice were inoculated into 35 mm plastic dish with 1 ml of MaCoy's 5A containinng 20% horse serum and 0.3% Bactoager. Cultures were stimulated by the additiona of a test sample with or without a conditioned medium of mouse spleen cells stimulated pockweed mitoger (PWM-SCM). After incubation for 6 to 7 days at 37°C in a fully humidified atmosphere of 5% CO$_2$ in air, cultures were mounted on the glass slides and dried, then fixed with 5% glutaraldehyde for 5 min and

4

stained for acetylcholinesterase. AchE staining solution is consisted of: 30 mg acetylthiocholine iodide, 45 · ml of 0.1 M sodium phosphte buffer, pH 6.0, 6 ml of 30 mM copper sulfate, 3 ml of 0.1 M sodium citrate and 6 ml of 5 mM potassium ferricianide. After staining, the slides were washed with Dulbeccoco's phasphate buffered saline (PBS) and counterstained in Harris' hematoxylin. A megakaryocyte colony was defined as a cluster of four or more acetycholinesterase positive cells.

(2) Plasma clot system

Meg-CSA were also measured using plasma clot system. 2.5 to 5.0 x $10^6$ bone marrow cells obtained from male C57B1/6N mice were added to a solution consisting of bovine embrionic extract, NCTC 109 with L-asparagine, NCTC 109, 10% bovine serum albumin (BSA), fetal calf serum (FCS) and a test sample with or without PWM-SCM and bovine plasma. 0.4 ml of this suspension was plased in the center of 35 mm plastic dish. After clotting 0.5 ml of alpher medium containing 10% FCS were added to the culture dish. After incubation for 6 to 7 days at 37°C in a fully humidified atomosphere of 5% $CO_2$ in air, the alpher medium with 10% FCS surrounding the plasma clot was removed and dehydrated by a piece of paper on its surface. AchE staining is carried out according to the same procedure described in the soft agar system.

(3) AChE activity assay method

Since AChE is contained in serum, this assay is carried out after inactivation of AChE present in serum by diisopropylfluorophosphate (DFP).

Bone marrow cells of male C57B1/6N mouse are suspended in Iscove's modified Dulbecco's medium (IMDM) at a concentration of 5 to 10 x $10^5$ cells/ml. To 100 $\mu$l of the suspension are added 60 $\mu$l of DFP-treated FCS and 40 $\mu$l of assay samples with or without PWM-SCM, and the mixture is put into a well of a 96 well-plate. The well is incubated in a $CO_2$ incubator at 37°C for 3 to 7 days.

After the incubation, to the well is added 50 $\mu$l of 1 M Tris phosphate buffer (pH 8.0) containing 0.265 M 5,5'-dithiobis-2-nitrobenzoic acid (DTNB) and 1% Triton X-100, and absorbance at 414 nm ($OD_{414}$) is measured. Next, 15 $\mu$l of 10 mM acetylcholine iodide solution is added, and after 30 to 60 minutes at a room temperature, the $OD_{414}$ is measured. The AChE activity is calculated from the difference between the two $OD_{414}$ measurements.

(b) Method of in vivo assay after administration to animal

(1) In vivo assay method-1

A sample is subcutaneously administered to a BDF$_1$ female 4 to 5 weeks old once, or once a day for seven days, and after the administration, the number of platelets in blood, and optionally, a ratio of AChE positive cells in femur bone marrow cells, is periodically determined. The number of platelets is measured by obtaining a blood sample from the heart and counting platelets under a phase-contrast microscope using a hemocytometer. The ratio of AChE positive cells is determined by flushing out bone marrow cells from the femur, preparing a single cell suspension, fixing the cells with 5% glutaraldehyde, counting nucleated cells, and then counting AChE positive cells after AChE staining.

(2) In vivo assay method-2

Female BDF1 mice (5 to 7 weeks old) were subcutaneously injected with 0.5 to 1.0 ml of a test sample for 5 days. 6 hours after the first injection or 24 hours after the last injection, blood samples were taken by vena cava inferior under anaesthesia and platelets were counted in a Micro Cell Counter CC180A (Toa Medical Electronics Co.). TPO activity was defined as the increase of the platelet number compared with vehicle injected mice.

(3) Measurement of newly formed platelets by $^{35}$S-prelabel method

As an indication of a formation of platelets, an uptake of $^{35}$S into platelets is measured according to a method of McDonald, T.P. et al. (McDonald, T.P. et al., Proc. Soc. EXP. Biol. Med. 144: 1006, 1973).

As an experimental animal, ICR or BDF$_1$ female mice, 4 to 5 weeks old, are used. An anti-mouse platelet serum prepared in rabbits is intravenously injected to the mouse, and on the fifth day and sixth day (the phase of rebound thrombocytosis) at intervals of 12 hours for a total of 4 times, a sample is subcutaneously administered to the abdomen. 12 hours after the final administration, $^{35}$S-sodium sulfate in 0.2 ml physiological saline is injected through a tail vein in an amount of about 20 μCi, and after a further 24 hours, 0.5 ml of blood sample (EDTA is added) is obtained from the vena cava inferior under ether anesthesia.

The number of peripheral platelets is determined by diluting 50,000-fold 10 μl of the blood sample, and counting platelets using a Coulter Counter®. The remaining blood sample is diluted with 2 ml of Dulbecco's phosphate buffered saline (PBS) supplemented with 1 mM EDTA, and the diluted blood is centrifuged at 1000 rpm for 5 minutes to obtain platelets rich plasma (PRP). 1.5 ml of the PRP is centrifuged at 3000 rpm for 10 minutes to obtain a platelet precipitate, which is then suspended in 1 ml of 0.85% ammonium chloride aqueous solution. The suspension is allowed to stand at a room temperature for 15 minutes to hemolyze erythrocytes, and the remaining platelets are twice washed with PBS with 1 mM EDTA, and the washed platelets are suspended in 0.5 ml of the same buffer. Then 50 μl of the platelet suspension is 20,000-fold diluted and the number of the washed platelets is determined by a Coulter Counter ®. Next, 0.4 ml of the platelet suspension is put into a vial, and 5 ml of Atomlight [R] (New England Nuclear) is added to count the radioactivity with a liquid scintillation counter.

From the measurement thus obtained, an uptake ratio of $^{35}$S-sodium sulfate into platelets is calculated as follows.

$$[\text{Uptake ratio of } ^{35}\text{S-sodium sulfate into platelets}]$$

$$= \frac{\text{Radioactivity of platelet suspension} \times 1/4}{\text{Number of washed platelets}}$$

$$\times \frac{\text{Number of peripheral platelets}}{\text{Radio activity of } ^{35}\text{S-input}} \times \text{total blood}^{(*)}$$

$$\times 100$$

$$(*\text{Total blood} = \text{body weight} \times 0.07)$$

Although the presence of TPO can be estimated by the above-described various methods, only the method (b)(2) can be used to detect directly the in vivo increase of platelets. Accordingly, the measurement of TPO activity was mainly carried out by this method. Note, the present inventors are not aware of any reports wherein such a direct in vivo method observed the increase of platelets.

TPO

The present inventors then proceeded to clarify a TPO in liposarcoma involving an increase of platelets. For this purpose, the present invention was started with the establishment of a liposalcoma cell line.

The established cell line of the present invention could be obtained from human liposarcoma cells, by a conventional procedure. The procedure used is described in detail in Example 1.

Although the cell line TNK-01 thus established produced TPO in Dulbecco's Modified Eagle Medium (DMEM) at early passaging, according to the passages proceeded, it had lost an ability to produce TPO. The present inventors, after various approaches, found that the TNK-01 cell line which has experienced many passages produces TPO when cultured in a medium containing 1 U/ml to 100 U/ml human interleukin-1α (IL-1α) or human interleukin-1β (IL-1β) which have been known to induce IL-6, and thus found the experimental base of the present invention.

According to the present invention, to produce TPO, an established animal cell line capable of TPO, such as TNK-01, is cultured in a conventional medium for culturing cells, such as DMEM, Minimum Essential Medium (MEM) or the like supplemented with a TPO inducer such as human IL-1α or IL-1β, in an appropriate atmosphere such as in 5% $CO_2$ in air, at a temperature of about 37°C for 4 to 8 days in an aerobic condition. During the culture a TPO is secreted in the culture supernatant.

The TPO can be recovered and purified by a conventional procedure used for the isolation and purification of a protein, such as ammonium sulfate fractionation, ultrafiltration, ion-exchange chromatography, high performance liquid chromatography and the like, alone or in combination. For example, the present TPO can be concentrated by ultra filtration using a membrane which passes materials having a molecular weight of up to about 5000. The concentrate can be subjected to an ion exchange chromatography such as DEAE-Sephorose CL-6B ion exchange chromatography to obtain a crude TPO fraction. This crude TPO fraction can be concentrated by ultrafiltration using a membrane having a cut-off value 5000. Moreover the concentrate can be fractionated by liquid chromatography using, for example, a TSK G3000 SW column, to obtain a fraction containing substances having a molecular weight of 20,000 to 40,000, including the present TPO. The active fraction thus obtained can be further purified by reverse phase high performance liquid chromatography, such as that using UYDAC 214 TP54, and eluting with an acetonitrile linear gradient containing 0.1% trifluoroacetic acid (TFA). The present TPO is eluted at acetonitrile concentration of 40 to 50%.

The thus-purified TPO is separated into a component having a molecular weight of 24,000 and a component having a molecular weight of 27,000 on SDS-polyacrylamide gel electrophoresis. The polypeptide having a molecular weight of 27,000 is expected to be a N-glycosylated form of the polypeptide having a molecular weight of 24,000. Namely, the components of the present TPO exhibits apparent molecular weights of about 24,000 and 27,000 as determined by SDS-polyacrylamide gel electrophoresis using lysozyme (molecular weight: 14,400), soy bean trypsin inhibitor (21,500), carbonic anhydrase (31,000)-,ovalbumin (45,000), calf serum albumin (66,200), and phosphorylase B (92,500) molecular weight standards as.

The present TPO purified as described above has an N-terminal amino acid sequence represented by the following sequence:

Val-Pro-Pro-Gly-Glu-Asp-Ser-Lys-Asp-Val-Ala-Ala-Pro-His-Arg-Gln-Pro-Leu-.

This amino acid sequence is identical to N-terminal amino acid sequence of known BSF2 (Van Damme, J. et al., Eur. J. Biochem., 168, 543-555, 1978; Hirano, T. et al., Nature 324. 73-76, 1986). Moreover, it was found that the present TPO, i.e., both the component having a molecular weight of 24,000 and the component having a molecular weight of 27,000, crossreacts with an antibody to BSF2 in Western blot analysis, revealing the identity of the present TPO with BSF2.

Moreover it was confirmed that known polypeptides having BSF2 activity also exhibit thrombopoietic activity.

Accordingly, as active ingredients of the present pharmaceutical preparation, in addition to the TPO produced by TNK-01 cell line, various kinds of polypeptides having BSF2 activity can be used.

In the present invention, polypeptides having BSF2 activity include any polypeptides which induce a B cell to produce antibodies, for example, native BSF2 described in Japanese Unexamined Patent Publication, KOKAI, Nos. 61-115204 and 61-115025; and polypeptides wherein one or more than one amino acid has been added to the N-terminal of the amino acid sequence (I), and polypeptides wherein one or more than one amino acid has been deleted from the N-terminal of the amino acid sequence (I) of a native type or produced by a gene recombination technique, described in Eur. J. Bichem. 168, 543-550 (1987). Moreover as polypeptides prepared by a gene recombination technique, EP-257406, EP-220574 and PCT International Publication No. WO88/00206 as well as BIO/TECHNOLOGY 6, 806-809, 1988 disclose polypeptides wherein one or more than one amino acid has been added to the N-terminal of the amino acid sequence (I), and Japanese Unexamined Patent Publication, KOKAI, No. 63-157996 discloses polypeptides wherein some amino acids have been deleted from the N-terminal of the amino acid sequence (I). Although typical polypeptides of the present invention are those having the above-mentioned various amino acid sequences and glycocylated derivatives thereof, other polypeptides having BSF2 activity, for example, polypeptide wherein one or more than one amino acid in the amino acid sequence (I) has been replaced by other amino acid(s), can be used.

The present polypeptides as described above can be produced according to corresponding procedure described in the above-mentioned references, and some of are commercially available from, for example, Amersham Japan, Boehringer Mannheim, Yamanouchi, Cosmo Bio, Genzyme, etc.

Polypeptides having BSF2 activity used as active ingredients in the present pharmaceutical preparation, or derivatives thereof, are endogeneous to the human body and therefore, there are few problems relating to side effects.

Diseases involving thrombocytopenia and/or impaired platelet functions include cancers, chronic hepatic disorders, renal disorders, severe infections, bone marrow hypoplasia, and the like.

The pharmaceutical preparation of the present invention is preferably administered subcutaneously or intravenously.

The application dose of a peptide having BSF2 activity depends on a specific activity of the particular polypeptide used, the disease to be treated, the condition of the individual, and other factors, and is usually 1 to 10,000 μg/day for an adult.

The present pharmaceutical preparation is prepared by, for example, dissolving a polypeptide having BSF2 activity in a conventional injective carrier such as physiological saline, aseptically filtrating the solution, and optionally, lyophilizing the filtrated solution.

Examples

The present invention will be further illustrated by, but is by no means limited to the following examples.

Example 1. Establishment of TPO producing cell line

(a) Tumor

A liposarcoma was aseptically removed from the left thigh of a patient having a remarkable increase of platelets, and sliced and cultured as follows:

(b) Primary culture

First, 10 to 15 tumor sections prepared as above were put into a 50 ml plastic centrifuge tube containing 5 ml of a trypsin solution containing 0.25% trypsin and 0.02% EDTA and were incubated at 37°C for 15 minutes. The supernatant was discarded, and 5 ml of the fresh trypsin solution was added, and trypsin digestion was carried out at 37°C for 15 minutes. The upper cell suspension was collected, and to this suspension was added 1 ml of FCS to terminate the trypsin digestion, and the suspension was stored on ice.

The above-described procedure was repeated to recover a cell suspension. The combined cell suspension was centrifuged at 1500 r.p.m. for 10 minutes to obtain a cell pellet. The cell pellet was washed twice with DMEM containing 10% FCS, and inoculated into a 25 cm$^2$ plastic culture flask at a cell concentration of 5 x 10$^6$ cells/flask. The cells were cultured in DMEM containing 10% in a $CO_2$ incubator overnight, and after removal of the supernatant, fresh DMEM containing 20% FCS was added to maintain the culture.

(c) Passage and establishment of cell line

When the cultured cells became confluent, the culture supernatant was removed, and the cells were washed two or three times with PBS and added 0.5 ml of trypsin solution. After incubation at 37°C for 5 minutes in a $CO_2$ incubator, a sufficient amount of DMEM containing FCS was added to recover the cells. The recovered cells were washed twice with DMEM containing 10% FCS, and one fifth to one tenth of the cells were inoculated to a fresh flask and passaged by culturing the cells in fresh DMEM containing 10% FCS. The passages were repeated to obtain a cell line able to stably grow, which was designated TNK-01.

The established cell line TNK-01 was deposited with the Fermentation Research Institute, Agency of Industrial and Technology, 1-3, Higashi 1-chome, Tsukuba-shi, Ibaraki-ken 305, Japan, on October 7, 1988 as FERM BP-2088, under the Budapest Treaty.

Example 2. Confirmation of TPO production at early stage of passages

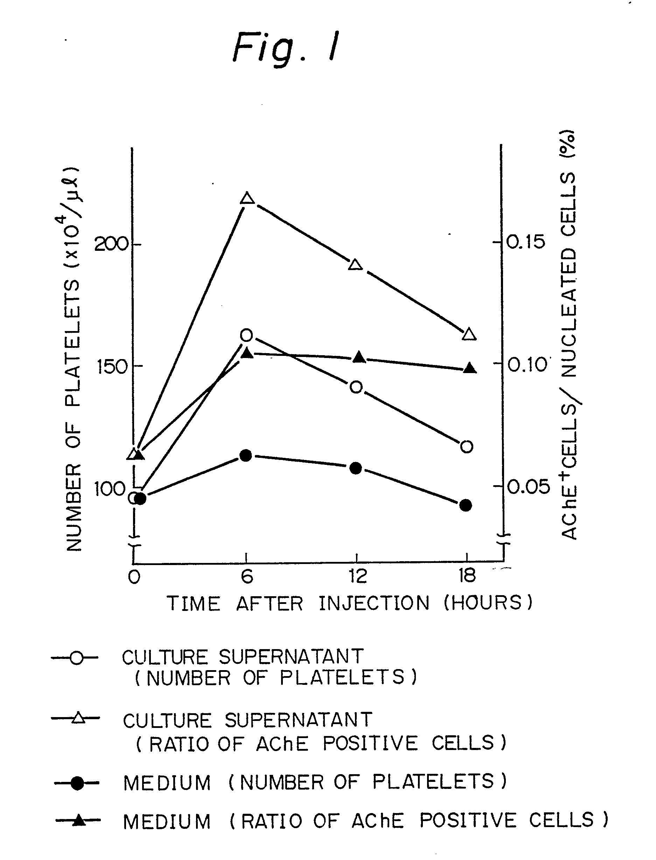

The TNK-01 cells were cultured in DMEM supplemented with 15% FCS at 37°C, and 1 ml of the culture supernatant subcutaneously injected to BDF$_1$ female mice 4 to 6 weeks old, and 6 hours, 12 hours, and 18 hours after the injection, blood samples were obtained and platelets were counted by the method (b)(1) described above using a hemocytometer. As a result, the number of platelets increased to 143%, 130%, and 126%, respectively, relative to the count before the injection. Moreover, the ratio of AChE positive cells relative to nucleated cells was as high as 257%, 221%, and 179%, respectively. In a parallel control test wherein 1 ml of the medium not inoculated with cells was injected in place of the culture supernatant, although the ratio of AChE positive cells relative to nucleated cells slightly increased, the number of platelets in the blood did not change. The result is summarized in Fig. 1.

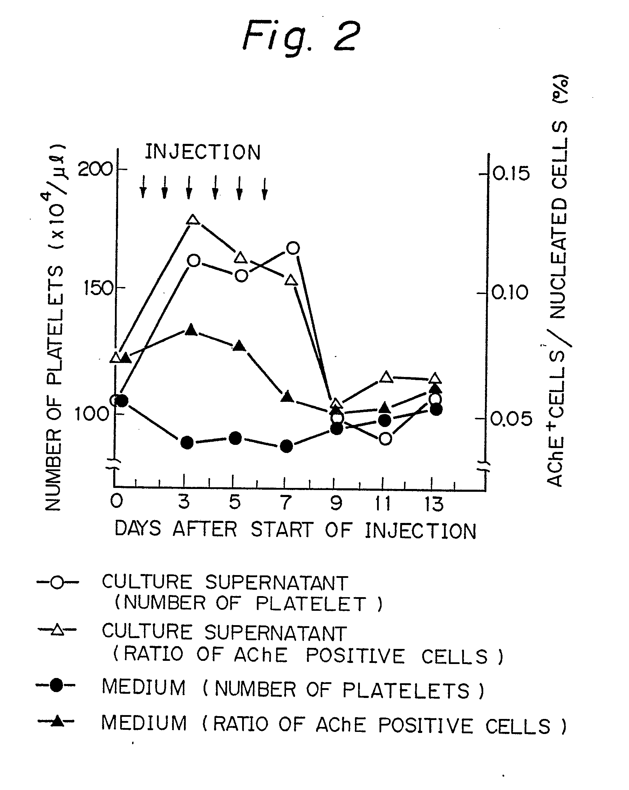

On the other hand, where 1 ml of the culture supernatant of TNK-01 cells was subcutaneously injected to mice once a day for 7 days, until one day after the final injection, the number of platelets was maintained at a level as high as 170 to 190% relative to a level before the first injection, and a ratio of AChE positive cells was also high. On the third day from the final injection, the number of platelets returned to a normal level. Again, in a control wherein 1 ml of the medium was injected in place of the culture supernatant, although a ratio of AChE positive cells slightly increased, the number of platelets did not increase. The result is shown in Fig. 2.

## Example 3. Purification of TPO from supernatant of TNK-01 culture at early stage of passages

The TNK-01 cells were cultured in DMEM supplemented with 15% FCS, to obtain a culture supernatant. The supernatant was fractionated by ammonium sulfate precipitation to obtain a fraction which precipitated at an ammonium sulfate 50% saturation (culture supernatant $(NH_4)_2SO_4$ 50% fraction), and a fraction which precipitated at an ammonium sulfate 50%-80% saturation (culture supernatant $(NH_4)_2SO_4$ 50%-80% fraction). As a control, the medium not inoculated with TNK-01 cells was treated as described above to obtain a "non-inoculated medium $(NH_4)_2SO_4$ 50% fraction", and a "non-inoculated medium $(NH_4)_2SO_4$ 50-80% fraction".

These fractions were dialyzed at a low temperature to eliminate ammonium sulfate, and assayed for the occurrence of AChE positive cells and the effect on AChE activity in an in vitro mouse bone marrow culture system, by the procedure as described above.

For the test, PWM-SCM alone, the above-mentioned "$(NH_4)_2SO_4$ fraction" alone, and a combination of PWM-SCM and the $(NH_4)_2SO_4$ fraction were used. In a test using the PWM-SCM alone and the $(NH_4)_2SO_4$ fraction alone, they were added at day 0 (at the onset of culture). In a test using the combination of PWM-SCM and the $(NH_4)_2SO_4$ fraction, the PWM-SCM was added at day 0 of the culturing, and the $(NH_4)_2SO_4$ fraction was added at day 0, day 3, or day 5 of the culture.

On the seventh day, the number of AChE positive cells and AChE activity in the culture were measured. The AChE activity was measured by the above-described method (a)(3), and the number of AChE positive cells was counted by staining cells cultured by the above-mentioned (a)(3), wherein the staining was carried out according to the above-mentioned (a)(1).

The results of the number of AChE positive cells are shown in Table 1; and the results of AChE activity are shown in Table 2.

Table 1

| Effect of fractions on the number of AChE positive cells | |
|---|---|
| Fraction tested | Number of AChE positive cells |
| PWM-SCM | 87 - 96 |
| Culture supernatant $(NH_4)_2SO_4$ 50% fraction | 18 |
| Non-inoculated medium $(NH_4)_2SO_4$ 50% fraction | 30 |
| Culture supernatant $(NH_4)_2SO_4$ 50-80% fraction | 48 |
| Non-inoculated medium $(NH_4)_2SO_4$ 50-80% fraction | 38 |
| PWM-SCM + Culture supernatant $(NH_4)_2SO_4$ 50% fraction (day 0) | 20 |
| PWM-SCM + Non-inoculated medium $(NH_4)_2SO_4$ 50% fraction (day 0) | 14 |
| PWM-SCM + culture supernatant $(NH_4)_2SO_4$ 50-80% fraction (day 0) | 144 |
| PWM-SCM + Non-inoculated medium $(NH_4)_2SO_4$ 50-80% fraction (day 0) | 92 |
| PWM-SCM + Culture supernatant $(NH_4)_2SO_4$ 50% fraction (day 3) | 12 |
| PWM-SCM + Non-inoculated medium $(NH_4)_2SO_4$ 50% fraction (day 3) | 16 |
| PWM-SCM + Culture supernatant $(NH_4)_2SO_4$ 50-80% fraction (day 3) | 64 |
| PWM-SCM + Non-inoculated medium $(NH_4)_2SO_4$ 50-80% fraction (day 3) | 56 |
| PWM-SCM + Culture supernatant $(NH_4)_2SO_4$ 50% fraction (day 5) | 52 |
| PWM-SCM + Non-inoculated medium $(NH_4)_2SO_4$ 50% fraction (day 5) | 24 |
| PWM-SCM + Culture supernatant $(NH_4)_2SO_4$ 50-80% fraction (day 5) | 44 |
| PWM-SCM + Non-inoculated medium $(NH_4)_2SO_4$ 50-80% fraction (day 5) | 70 |

Table 2

| Effect of fractions on AChE activity | |
|---|---|
| Fraction tested | AChE activity |
| PWM-SCM | 0.30-0.49 |
| Culture supernatant $(NH_4)_2SO_4$ 50% fraction | 0.20 |
| Non-inoculated medium $(NH_4)_2SO_4$ 50% fraction | 0.20 |
| Culture supernatant $(NH_4)_2SO_4$ 50-80% fraction | 0.52 |
| Non-inoculated medium $(NH_4)_2SO_4$ 50-80% fraction | 0.28 |
| PWM-SCM + Culture supernatant $(NH_4)_2SO_4$ 50% fraction (day 0) | 0.25 |
| PWM-SCM + Non-inoculated medium $(NH_4)_2SO_4$ 50% fraction (day 0) | 0.10 |
| PWM-SCM + culture supernatant $(NH_4)_2SO_4$ 50-80% fraction (day 0) | 0.48 |
| PWM-SCM + Non-inoculated medium $(NH_4)_2SO_4$ 50-80% fraction (day 0) | 0.28 |
| PWM-SCM + Culture supernatant $(NH_4)_2SO_4$ 50% fraction (day 3) | 0.27 |
| PWM-SCM + Non-inoculated medium $(NH_4)_2SO_4$ 50% fraction (day 3) | 0.17 |
| PWM-SCM + Culture supernatant $(NH_4)_2SO_4$ 50-80% fraction (day 3) | 0.44 |
| PWM-SCM + Non-inoculated medium $(NH_4)_2SO_4$ 50-80% fraction (day 3) | 0.29 |
| PWM-SCM + Culture supernatant $(NH_4)_2SO_4$ 50% fraction (day 5) | 0.38 |
| PWM-SCM + Non-inoculated medium $(NH_4)_2SO_4$ 50% fraction (day 5) | 0.20 |
| PWM-SCM + Culture supernatant $(NH_4)_2SO_4$ 50-80% fraction (day 5) | 0.37 |
| PWM-SCM + Non-inoculated medium $(NH_4)_2SO_4$ 50-80% fraction (day 5) | 0.37 |

As seen from the above results, both activities of the culture product of increasing the number of AChE positive cells and increasing the AChE activity ($OD_{414}$) are present in the $(NH_4)_2SO_4$ 50-80% fractions, and these activities are enhanced by the presence of PWM-SCM.

## Example 4. TPO production by TNK-01 cells after long-term passages

### (a) Decrease of TPO productivity of TNK-01 cells after long-term passages

According to the passages proceeded, the thrombopoietic activity of culture supernatant of TNK-01 decreased, suggesting a decrease of the TPO productivity of the TNK-01 cell line. But the TNK-01 cell line whose TPO productivity had decreased still produced the potentiating activity for megakaryocyte maturation in the culture supernatant, in an in vitro assay using mouse bone marrow culture.

### (b) Induction of TPO production by various stimulating substances

To induce the TPO production, TNK-01 cells were initially grown in DMEM containing 20% FCS. After the cells reached confluence, the spent medium was replaced with fresh DMEM containing 20% FCS and an appropriate concentration of reagent such as hydrocortisone, 12-0-tetradecanonyl phorbor 13-actate (TPA), human recombinant IL-1$\alpha$ or human recombinant IL-1$\beta$. After 4 days cultivation, the supernatants were pooled and stored at -20$^\circ$C until use.

1 ml of the supernatant was subcutaneously injected to female BDF1 mice and 6 hours after the injection, blood samples were taken by vena cava inferior under anaesthesia and platelets were counted in a Micro Cell Counter CC180A (schedule 1). In the schedule 2, 1 ml of the supernatant was subcutaneously injected to the mice for five days and 24 hours after the last injection, blood sample was obtained and platelets were counted as previously described. As controls, the medium not inoculated with TNK-01 cells, and a culture supernatant obtained by culturing TNK-01 cells in the medium not containing inducer candidate were used. In the Tables, the number of platelets (average number ± standard error) is shown by x $10^4/\mu$l.

The results were shown in Table 3 to 6. Hydrocortisone and TPA were not induce TPO from TNK-01 cell line. But IL-1$\alpha$ or IL-1$\beta$ were significantly induced TPO.

Table 3

| Induction of TPO production by hydrocortisone | | |
|---|---|---|
| Sample | Schedule 1 | Schedule 2 |
| Control (medium) | - | 106.2 ± 4.9 |
| Culture supernatant ( 0 M)[*] | 114.0 ± 1.0 | 109.1 ± 4.6 |
| Culture supernatant ($10^{-7}$ M) | 110.6 ± 2.3 | 104.6 ± 2.5 |
| Culture supernatant ($10^{-6}$ M) | 116.0 ± 4.6 | 112.2 ± 4.2 |
| Culture supernatant ($10^{-5}$ M) | 109.2 ± 3.3 | 101.1 ± 1.2 |

[*] Parenthesized numerical values are concentration of hydrocortisone added.

Table 4

| Induction of TPO production by 12-O-tetradecanoyl phorbor 13-acetate (TPA) | |
|---|---|
| Sample | Schedule 2 |
| Culture supernatant ( 0 M)[*] | 92.9 ± 5.3 |
| Culture supernatant ($10^{-8}$ M) | 99.2 ± 2.0 |
| Culture supernatant ($10^{-7}$ M) | 104.7 ± 2.4 |
| Culture supernatant ($10^{-6}$ M) | 102.9 ± 3.5 |

[*] Parenthesized numerical values are concentration of TPA added.

Table 5

| Induction of TPO production by IL-1$\alpha$ | | |
|---|---|---|
| Sample | Schedule 1 | Schedule 2 |
| Control (medium) | - | 106.2 ± 4.9 |
| Culture supernatant ( 0 U/ml)[*] | 114.0 ± 1.0 | 109.1 ± 4.6 |
| Culture supernatant ( 1 U/ml) | 115.7 ± 6.4 | 127.0 ± 7.4 |
| Culture supernatant ( 10 U/ml) | 104.0 ± 2.7 | 128.0 ± 3.5[**] |
| Culture supernatant (100 U/ml) | 102.8 ± 2.6 | 125.4 ± 3.4[**] |

[*] Parenthesized numerical values are concentration of IL-1$\alpha$ added.
[**] Significant difference by $P < 0.05$

Table 6

| Induction of TPO production by IL-1$\beta$ | |
|---|---|
| Sample | Schedule 2 |
| Control (medium) | 104.4 ± 5.7 |
| Medium + 1L-1$\beta$ (10 U/ml) | 106.2 ± 1.5 |
| Culture supernatant (IL-1$\beta$ not added) | 97.1 ± 2.2 |
| Culture supernatant (IL-1$\beta$ 10 U/ml) | 122.7 ± 3.9[**] |

[**] Significant difference by $P < 0.05$

(c) Confirmation of necessity for induction of TPO production by IL-1$\alpha$ or IL-1$\beta$

Since it was found in Example 4 (b) that an induction by human IL-1$\alpha$ or IL-1$\beta$ is essential for a TPO production by TNK-01 cells, the necessity for induction was further confirmed for IL-1$\alpha$.

TNK-01 cells were cultured in DMEM supplemented with 20% FCS, and the supernatant was replaced by DMEM supplemented with 10 U/ml IL-1$\alpha$ and 2% FCS at 4 day intervals, and the TPO activity in the culture supernatants was assayed. In another experiment, the supernatant was alternately replaced by the above-mentioned medium containing 10 U/ml IL-1$\alpha$ and the same medium excluding IL-1$\alpha$, and the TPO activity in the culture supernatant was assayed. The TPO activity was assayed by measuring the increase of platelets according to the above described method (b) (2).

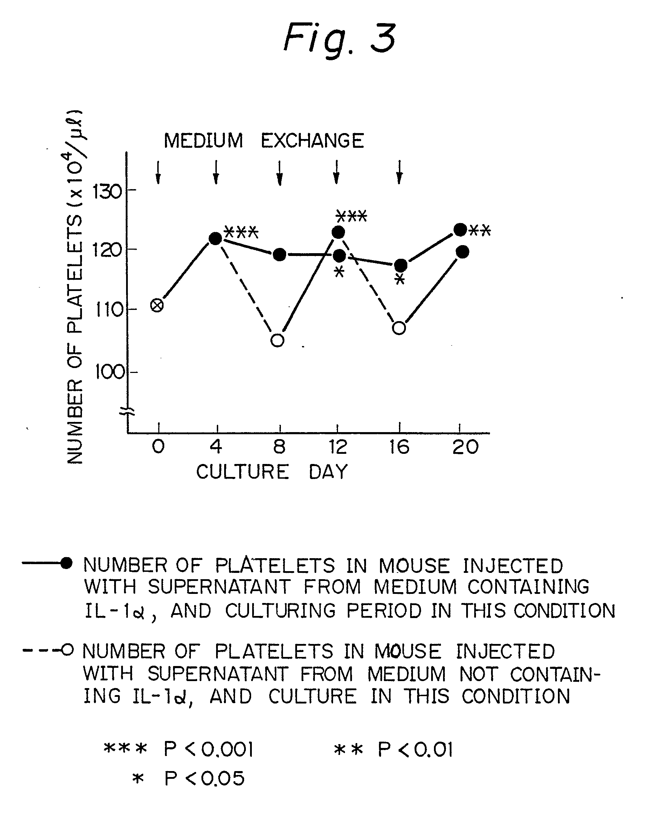

The results are shown in Fig. 3. In Fig. 3, the numbers of platelets are plotted along the culture time (days). The adjacent plots are linked by a solid line whereat the medium contained IL-1α during corresponding period, and the adjacent plates are linked by a dotted line whereat the medium did not contain IL-1α during the corresponding period. From Fig 3, it is seen that the number of platelets was continually maintained at a high level when used the supernatants cultured in the medium containing IL-1α during the corresponding period. On the other hand, where the IL-1α-containing medium and the IL-1α-free medium were alternately used, although supernatants from the IL-1α-containing medium increased the number of platelets in mice, supernatants from the IL-1α free medium did not, and therefore, it was concluded that the TPO production by TNK-01 cells requires an inducer such as IL-1α in a culture medium.

Example 5. Behavior upon ultrafiltration of TPO activity in TNK-01 culture supernatant prepared under IL-1α induction

(a) Concentration

TNK-01 cells were initally grown in DMEM containing 20% FCS replaced with fresh DMEM containing 20% FCS and 10U/ml of IL-1α. After 4 days cultivation, the supernatants were pooled and concentrated by ultrafiltration using Amicon PM-5 membrance.

(b) TPO of activity in the concentrate

To test the behavior of the TPO activity upon ultrafiltration, (1) 1 ml of the medium not inoculated with TNK-01, (2) 1 ml of the medium not inoculated with TNK-01 but supplemented with 10 U/ml IL-1α, (3) 1 ml of the culture supernatant, (4) 0.2 ml of the Amicon concentrate from the culture supernatant, and (5) 1 ml of the Amicon concentrate from the culture supernatant, were used to count the number of platelets by the method above-described (b) (2). The results are shown in Table 7.

Table 7

| Sample | Administration dose (ml) | Number of platelets (x $10^4/\mu l$) |
|---|---|---|
| Medium | 1.0 | 111.5 ± 1.6 |
| Medium + 1L - 1α | 1.0 | 120.8 ± 3.2[*] |
| Culture supernatant | 1.0 | 140.8 ± 4.7[**] |
| Concentrated culture supernatant | 0.2 | 140.7 ± 4.5[***] |
| Concentrated culture | 1.0 | 153.7 ± 7.3[**] |
| Number of platelets: mean ± standard error | | |

[*] $P < 0.05$
[**] $P < 0.01$
[***] $P < 0.001$

As seen from Table 7, since the administration of 1 ml of the supernatant and the administration of 0.2 ml of the 5-fold concentrated supernatant provided a comparable increase of platelets, and the molecular weight cut-off value of the used membrane was 5000, it is estimated that molecular weight of a substance having TPO activity in the culture supernatant is more than 5000.

Example 6. Confirmation of presence of TPO activity in TNK-01 culture supernatant prepared under IL-1α induction (1)

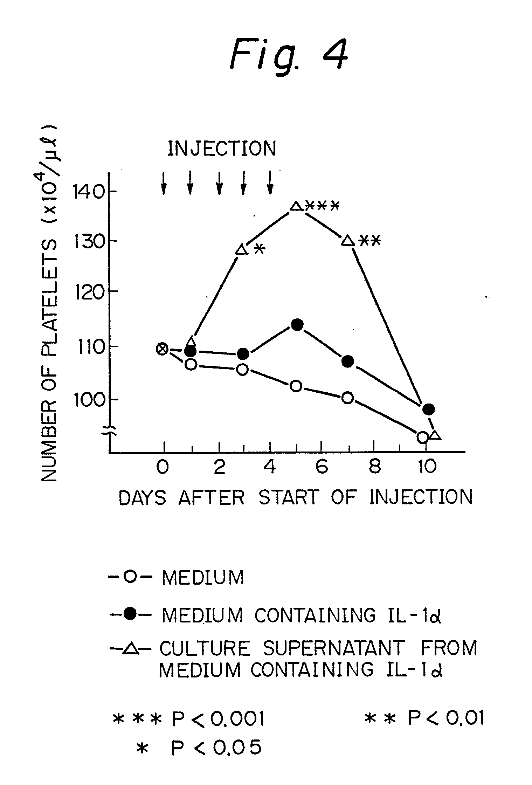

TNK-01 cells were cultured in DMEM supplemented with 20% FCS and 10 U/ml human IL-1α to obtain the supernatant. As controls, the above medium, and a medium excluding IL-1a, both not inoculated with TNK-01 cells, were used. These samples were subcutaneously injected to a BDF$_1$ female mice, 5 weeks old, successively for 5 days, and blood samples were obtained before the first injection, as well as 1, 3, 5, 7, and 10 days after the first injection. For each blood sample, the number of platelets was counted using a Micro Cell Counter CC180A. The results are shown in Fig. 4. As seen from the Figure, only the TNK-01 culture supernatant from the IL-1$\alpha$ containing medium significantly increased the number of platelets.

Example 7. Confirmation of presence of TPO activity in TNK-01 culture supernatant prepared under IL-1$\alpha$ induction (2)

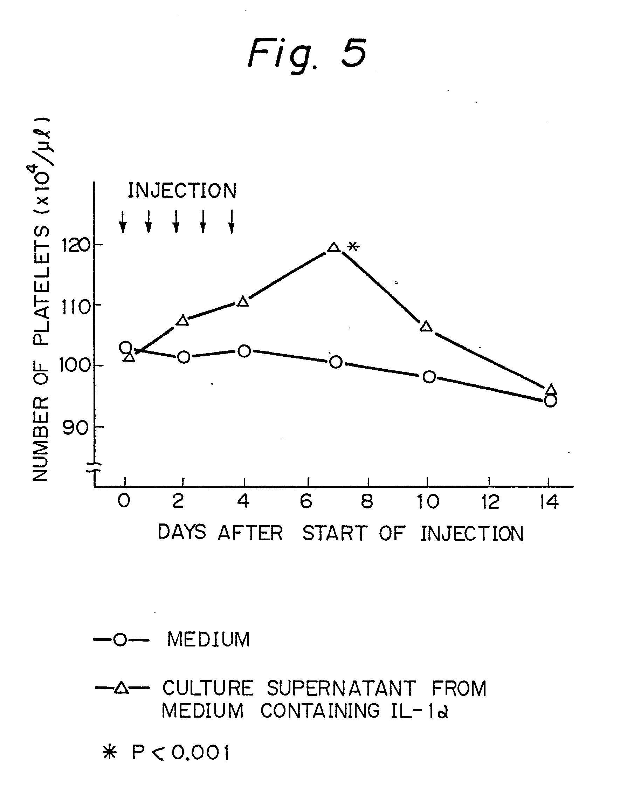

TNK-01 cells were cultured in DMEM supplemented with 20% and 10 U/ml human IL-1$\alpha$ to obtain the culture supernatant. As a control, the above-described medium not inoculated with TNK-01 cells was used. These samples were subcutaneously injected to SD male rats 9 weeks old, and blood samples were obtained before the first injection, as well as 2, 4, 7, 10 and 14 days after the first injection, through a tail vein, and the number of platelets was counted using a Micro Cell Counter CC180A. The results are shown in Fig. 5. As seen from Fig. 5, where the TNK-01 culture supernatant was injected, a tendency was observed toward an increase of platelets on the second and fourth days after the first injection, and a significant increase of platelets was observed on the seventh day.

Example 8. Behavior of TPO activity upon high performance liquid chromatography

The behavior of the TPO activity upon high performance liquid chromatography (HPLC) was studied. using two columns having different separation modes. The experiments in this Example were carried out at a room temperature (about 25° C).

(a) Molecular weight estimation of TPO using TSK G3000 SW column

Long-term passaged TNK-01 cells were cultured in DMEM supplemented with 2% FCS and 10 U/ml human IL-1$\alpha$ for 7 days to obtain the culture supernatant, which was then 20-fold concentrated by ultrafiltration using Amicon PM-5 membrane(Amicon) (molecular weight cut-off: 5000).

As molecular weight standards, calf serum albumin (molecular weight: 67,000), ovalbumin (43,000), $\beta$-lactoglobulin (36,800), chymotrypsynogen (25,000), and cytochrome C (12,300) were used. The standards and the sample were applied separately to a TSK G3000 SW column (21.5 mm x 600 mm) equilibrated with 50 mM sodium phosphate buffer (pH 7.4) containing 0.15 M NaCl and 0.05% Tween 20, and eluted with the same buffer at a flow rate of 3.75 ml/min.

The retention time was determined by following the absorbance at 280 nm for the molecular weight standards, and measuring the TPO activity in fractions by the method (b) (2). The molecular weight of the TPO was estimated to be 20,000 to 40,000 by comparing a retention time of the TPO activity and a calibration curve obtained from retention times of the molecular weight standards. An elution profile is shown in Fig. 6.

(b) Reverse HPLC using VYDAC 214 TP54 column

A TPO active fractions, obtained from HPLC using a TSK G3000 SW column as described in Example 8 (a), was concentrated about 20-folds by ultrafiltration using Amicon PM-10 (Amicon) (molecular weight cut-off: 10,000) to obtain a test sample.

The sample was applied to a reverse phase HPLC column, VYDAC 214 TP54 (C4, 4.6 x 250 mm) equilibrated with 5% acetonitrile aqueous solution containing 0.1% trifluoroacetic acid. Proteins adsorved to the column were eluted by a linear gradient of acetonitrile of 5% to 80% in 0.1% trifluoroacetic acid at a flow rate of 1 ml/min. The TPO activity was measured according to the method (b) (2). As a result, it was found that the TPO was adsorved on a VYDAC 214 TP54 reverse column, and eluted at an acetonitrile concentration of 40 to 50%. From this result, it is considered that the TPO is relatively hydrophobic.

Example 9. Purification of TPO from TNK-01 culture supernatant prepared under IL-1α induction

Step 1: DEAE Sepharose CL-6B ion-exchange chromatography

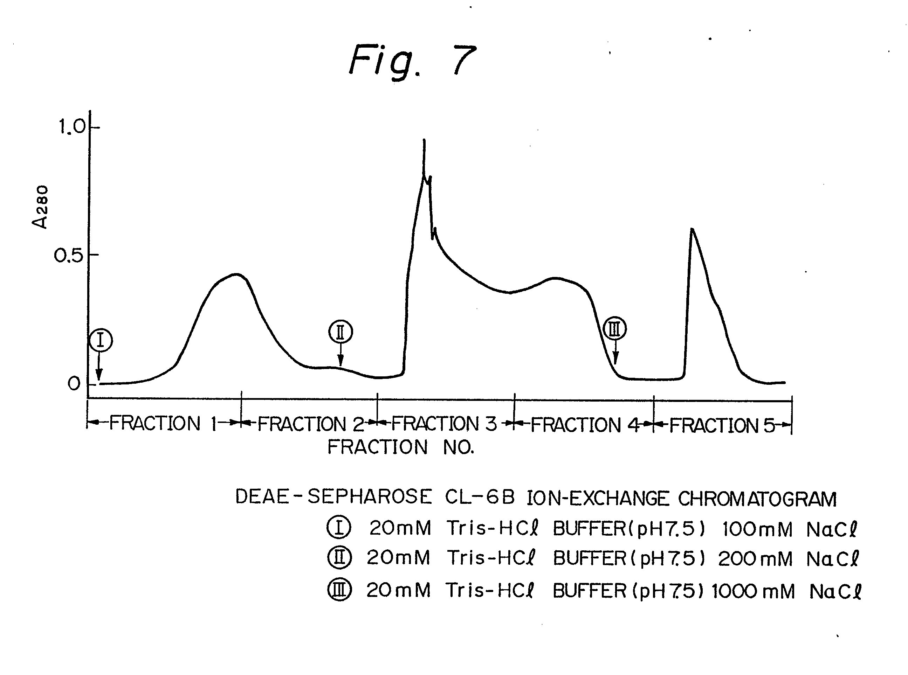

TNK-01 cells were cultured in DMEM supplemented with 2% FCS and 10 U/ml IL-1α for 7 days to obtain the culture supernatant, which was then concentrated 20-fold by ultrafiltration using Amicon PM-10 membrane (Amicon). The concentrate was dialyzed against 20 mM Tris-HCl (pH 7.5) buffer containing 0.1 M NaCl at 4°C for a night. The dialyzate (internal solution) was applied to a DEAE-Sepharose CL-6B (Pharmacia) column (2.2 x 22 cm) equilibrated with the above buffer, and proteins were successively eluted with 20 mM Tris-HCl (pH 7.5) buffer containing 0.1, 0.2 and 1 M NaCl. Each fraction was assayed for TPO activity by the method (b) (2). The TPO activity was detected in a latter half of a flow through fraction (fraction 2). The results are shown in Fig. 7.

Step 2: TSK G3000 SW gel-permeation chromatography

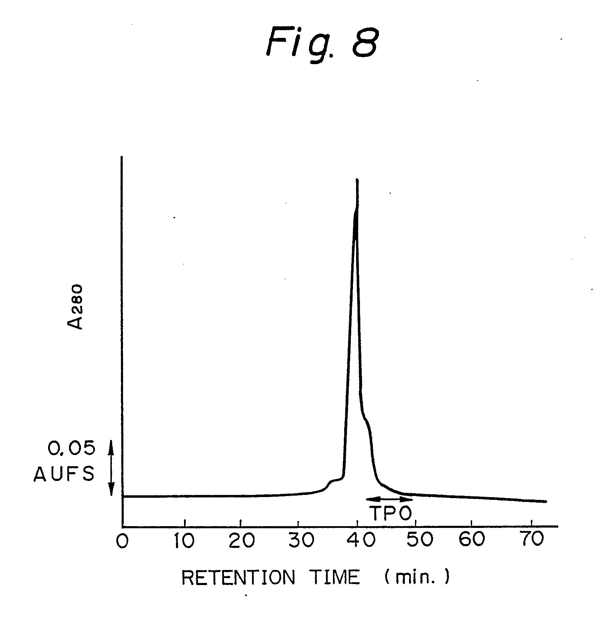

The TPO active fraction obtained from step 1 was concentrated 30-folds using Amicon PM-10 (Amicon), and the concentrate was applied to a TSK-G3000 SW column (21.5 x 600 cm) and eluted with the same buffer at a flow rate of 3.75 ml/min. The TPO activity was measured by the method (b) (2). The TPO was eluted at a retention time of 43 to 49 minutes. The elution profile is shown in Fig. 8. In the figure, a solid line represents the absorbance at 280 nm; and the TPO fraction is shown by an arrow.

Step 3: Reverse phase HPLC using Vydac C4 column

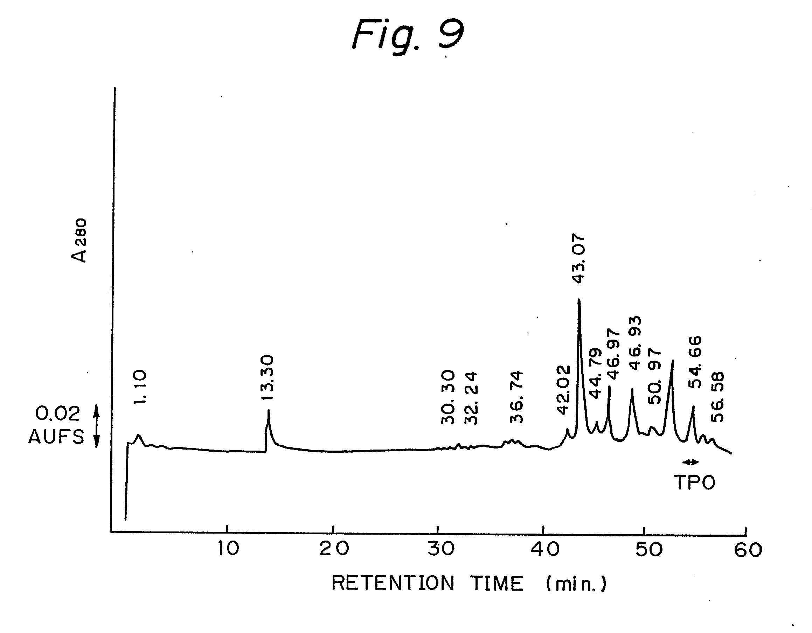

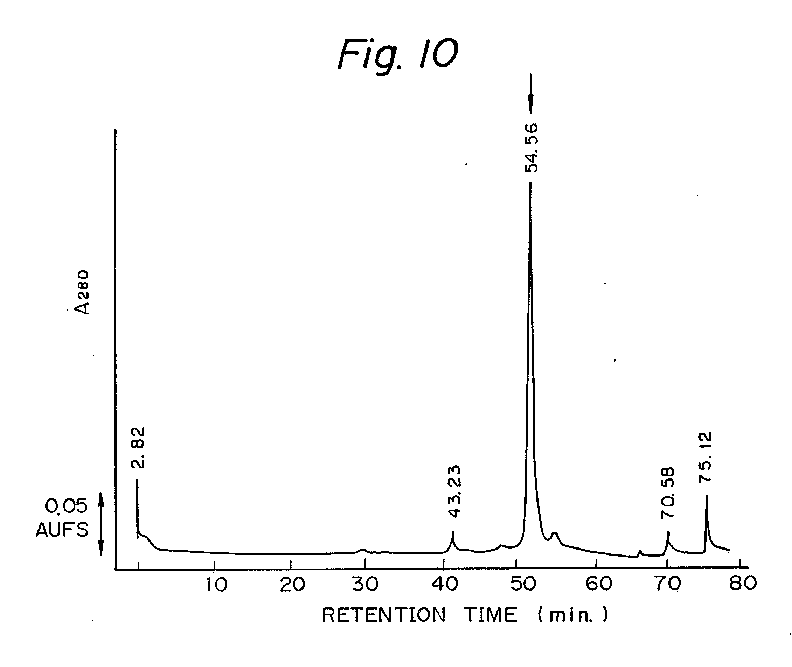

The TPO active fraction obtained from step 2 was subjected to reverse-HPLC using a Vydac C4 column. The column (4.6 x 250 mm) was equilibrated with 5% acetonitrile aqueous solution containing 0.1% trifluoroacetic acid, and after applying the active fraction, proteins absorbed to the column were eluted by a linear gradient of acetonitrile from 5% to 80% over 75 minutes. The TPO activity in the fraction was assayed by the method (b) (2). As seen from Fig. 9, TPO was eluted at retention time of 54 to 55 minutes. This TPO fraction was subjected to Vydac C4 reverse phase HPLC under the same conditions as described above to obtain purified TPO. The elution profile is shown in Fig. 10.

Example 10. Characterization of purified TPO

(1) Determination of N-terminal amino acid sequence of purified TPO

The N-terminal amino acid sequence of the purified TPO obtained by Example 9 was determined using an on-line system of a gas phase protein sequencer 470A and a PTH analyzer 120A (Applied Biosystems Inc.), as follows:

<p style="text-align:center">1         5          10<br>Val-Pro-Pro-Gly-Glu-Asp-Ser-Lys-Asp-Val-</p>

<p style="text-align:center">15<br>Ala-Ala-Pro-His-Arg-Gln-Pro-Leu-</p>

(2) Estimation of molecular weight of purified TPO

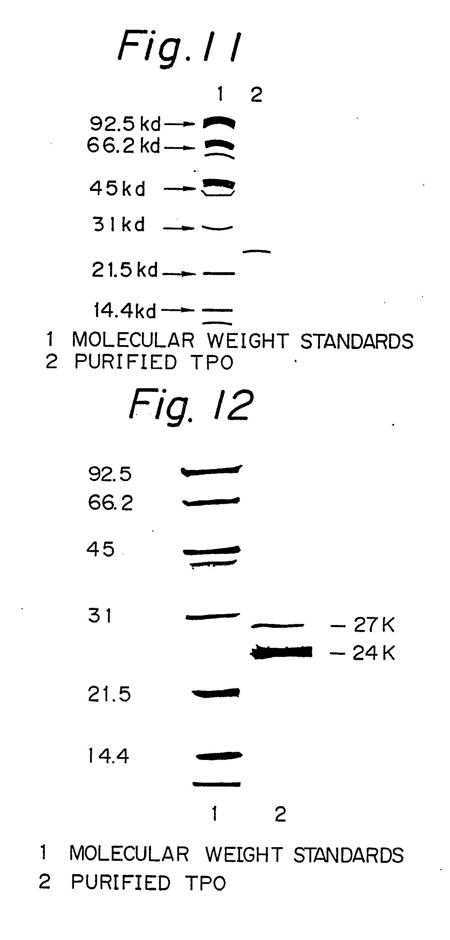

The molecular weight of purified TPO was estimated as about 24,000, by SDS-polyacrylamide gel electrophoresis (13.5% gel, 0.1% SDS) using as molecular weight standards lysozyme (molecular weight: 14,000), soy bean trypsin inhibitor (21,500), carbonic anhydrase (31,000) ovalbumin (45,000), bovine serum albumin (66,200) and phosphorylase B (92,500). The results are shown in Fig. 11. Note, the above electrophoresis was carried out at 100 V for 96 minutes, and the detection was carried out by staining with 0.1% Coomassie Blue R250.

On the other hand, where the above-mentioned electrophoresis was followed by development using an antibody to BSF2, a major component having a molecular weight of about 24,000 and a minor component having a molecular weight of about 27,000 were detected as shown in Fig. 12.

Experiment 1

The thrombopoietic activity of TPO was confirmed as follows.

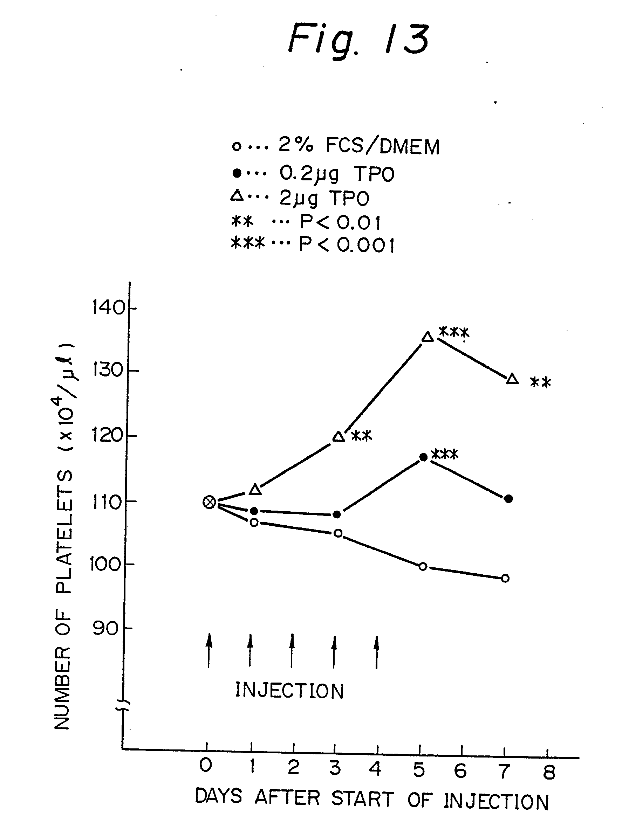

The purified TPO prepared in Example 9 was diluted with DMEM containing 2% FCS. 0.5 ml of appropriated concentration of the TPO or vehicle were subcutaneously injected to female BDF1 mice (from Charles River) for five days. Blood samples were obtained from vena cava inferior under anaesthesia on day 0, 1, 3, 5, 7, and platelets were counted in a Micro Cell Counter CC180A (Toa Medical Electronics Co.). The results were shown in Fig. 13.

As shown in Fig. 13, the purified TPO significantly increased the number of platelets in vivo in mice.

Experiment 2

Female BDF1 mice (five weeks old, purchased from Japan Charles River) were used in this experiment.

Ala-BSF2 (polypeptide having an additional alanine attached to the N-terminal of the sequence (I)) was prepared according to a procedure as described in BIO/TECHNOLOGY 6, 1988 and was diluted with a DMEM containing 2% FCS. 0.5 ml of appropriate concentration of Ala-BSF2 or vehicle were subcutaneously injected to the mice. Blood sample was obtained from vena cava inferior using a syringe containing 20 $\mu$l of 77 mM EDTA under anaesthesia on day 0, 1, 3, 5, 7, and platelets were counted in a Micro Cell Counter CC180A (Toa Medical Electronics Co.). The results were shown in Fig. 14.

As shown in Fig. 14, human recombinant BSF2 significantly increased the number of platelets in vivo in mice.

Experiment 3.

Female BDF1 mice were used in this experiment. Human recombinant interleukin-6 (IL-6; purchased from Amersham) was diluted DMEM containing 2% FCS. An appropriate concentration of the IL-6 or vehicle were subcutaneously injected to the mice for five days. 24 hours after the last injection blood sample was obtained from vena cava inferior under anaesthesia and platelets were counted in a Micro Cell Counter CC180A (Toa Medical Electronics Co.).

As a results, the number of platelets was $92.5 \pm 1.9 \times 10^4/mm^3$ for the vehicle injected mice, but as high as $131.9 \pm 3.2 \times 10^4/mm^3$ for the IL-6 injected mice.

Experiment 4. Effect on megakaryocyte colony formation

1) Preparation of bone marrow cell suspension
Femur myelocytes of a BDF1 female mouse were suspended in IMDM containing 1% bovine serum albumin and 1% Nutridoma SP (Boehringer Mannheim) at a concentration of $1 \times 10^6$ cells/ml.

2) Sample
Human recombinant BSF2 (Genzyme, Code No. HIL-6, specific activity: $10^7$ U/mg; one U is an amount necessary to increase immunoglobulin produced by CESS cells by 50% of maximum production) was diluted with the medium to prepare 40, 200 and 1000 U/ml solutions.

3) Culturing
The following mixtures: (1) 250 $\mu$l of the bone marrow cell suspension ($1 \times 10^6$ cells/ml), (2) 250 $\mu$l of the sample prepared in the above 2), (3) 250 $\mu$l of alpha medium containing 1% bovine serum albumin and 1% Nutridoma SP, (4) 500 $\mu$l of IMDM containing 1% Nutridoma SP, (5) 25 $\mu$l of $10^{-2}$ M 2-mercaptoethanol, and (6) 1000 $\mu$l of IMDM containing 7.5 mg/ml agar and 1% Nutridoma SP, were prepared and the mixtures (1) to (6) were mixed to prepare 1 ml of a mixture. This mixture was put into a Corning 35 mm dish, which was then incubated at 37°C and a relative humidity of 100% in 5% $CO_2$, for 6 days.

4) Count of megakaryocyte colonies

After the incubation, the agar was transferred to a slide glass, and fixed by a 10 mM phosphate buffer (pH 7.4) containing 5% glutaraldehyde. The cells were stained with acethyl choline esterase (AChE) and microscopically observed. A cell cluster consisting of at least 4 AChE positive cells was considered a megakaryocyte colony.

The results are shown in Fig. 15.

As seen from Fig. 15, BSF2 significantly stimulated the megakaryocyte colony formation.

Experiment 5.

1) Animal

BDF1 female mice (Nippon Charles River) 5 weeks old, were used.

2) Sample

BSF2 used in Experiment 4. 2) was diluted with DMEM containing 2% FCS (dilution medium) to prepare 20, 200, 2000, and 20000 U/ml solutions. As a control, the dilution medium was used.

3) Administration

0.5 ml of the solution prepared in the paragraph 2) was subcutaneously injected to 4 animals described in 3) once a day sequentially for 5 days.

4) Taking blood sample and counting platelets

24 hours after the final injection, 0.4 ml of a blood sample was obtained by a syringe containing 20 $\mu$1 of 77 mM EDTA from the vena cava inferior under ether anesthesia. The number of platelets was counted using a microcounter CC180A (Sysmex).

The results are shown in Table 8. An administration of BSF2 at a dose of 1000 U/head/day and 10000 U/head/day significantly increased the number of platelets.

Table 8

| Amount of BSF2 (U/head) | Number of platelets $(\times 10^4/mm^3)$ |
|---|---|
| 0 (control) | 113.0 ± 5.9 |
| 10 | 106 ± 1.8 |
| 100 | 117 ± 4.5 |
| 1000 | 130 ± 4.2* |
| 10000 | 136 ± 2.2** |

\* $P < 0.05$
\*\* $P < 0.01$

Formulation 1.

The TPO purified according to Example 9 was dissolved in physiological saline, and was aseptically filtered, the solution was distributed to ampoules for injection.

Formulation 2.

Purified BSF2 (Amgen, human recombinant IL-6, Product code: ARN 20025; sold by Amersham) was dissolved in physiological saline, the solution was aseptically filtered, and each of the solution was distributed to ampoules for injection.

**Claims**

1. A pharmaceutical preparation containing as an active ingredient a polypeptide having an activity of a human B cell stimulating factor, for a treatment of diseases involving thrombocytopenia or impaired platelet functions.

2. A pharmaceutical preparation according to claim 1, wherein the polypeptide is a polypeptide prepared by culturing a TNK-01 cell line established from human liposarcoma, and having the following properties:

(1) having an apparent molecular weight of about 24,000 or 27,000 as determined by SDS-polyacrylamide gel electrophoresis using as molecular weight standards lysozyme (molecular weight 14,400), soy bean trypsin inhibitor (21,500), carbonic anhydrase (31,000), ovalbumin (45,000), bovine serum albumin (66,200) and phosphorylase B (92,500); and

(2) having the following N-terminal amino acid sequence:

(N terminal) Val-Pro-Pro-Gly-Glu-Asp-Ser-Lys-Asp-Val-Ala-Ala-Pro-His-Arg-Gln-Pro-Leu-.

4. A pharmaceutical preparation according to claim 1, wherein the polypeptide is a polypeptide having the following amino acid sequence (I):

Pro Val Pro Pro Gly Glu Asp Ser Lys Asp Val Ala Ala Pro His Arg Gln Pro Leu Thr Ser Ser Glu Arg Ile Asp Lys Gln Ile Arg Tyr Ile Leu Asp Gly Ile Ser Ala Leu Arg Lys Glu Thr Cys Asn Lys Ser Asn Met Cys Glu Ser Ser Lys Glu Ala Leu Ala Glu Asn Asn Leu Asn Leu Pro Lys Met Ala Glu Lys Asp Gly Cys Phe Gln Ser Gly Phe Asn Glu Glu Thr Cys Leu Val Lys Ile Ile Thr Gly Leu Leu Glu Phe Glu Val Tyr Leu Glu Tyr Leu Gln Asn Arg Phe Glu Ser Ser Glu Glu Gln Ala Arg Ala Val Gln Met Ser Thr Lys Val Leu Ile Gln Phe Leu Gln Lys Lys Ala Lys Asn Leu Asp Ala Ile Thr Thr Pro Asp Pro Thr Thr Asn Ala Ser Leu Leu Thr Lys Leu Gln Ala Gln Asn Gln Trp Leu Gln Asp Met Thr Thr His Leu Ile Leu Arg Ser Phe Lys Glu Phe Leu Gln Ser Ser Leu Arg Ala Leu Arg Gln Met.

6. A pharmaceutical preparation according to claim 4, wherein the polypeptide is a polypeptide which has the amino acid sequence represented by formula (I) and has been glycosylated.

7. A pharmaceutical preparation according to claim 4, wherein the polypeptide is a polypeptide wherein one or more than one amino acid has been added to a N-terminal or C-terminal or both terminals, of the amino acid sequence represented by the formula (I).

8. A pharmaceutical preparation according to claim 4, wherein the polypeptide is a polypeptide wherein one or more than one amino acid has been deleted N-, C-terminal from the amino acid sequence represented by the formula (I).

9. A pharmaceutical preparation according to claim 4, wherein the polypeptide is a polypeptide wherein one or more than one amino acid present in the amino acid sequence represented by the formula (I) has been replaced by one or more than one other amino acid.

10. The use of a polypeptide having an activity of a human B cell stimulating factor for producing a pharmaceutical preparation for a treatment of diseases involving thrombocytopenia or impaired platelet functions.

11. A cell line TNK-01 (FERM BP-2088) or mutants and derivatives thereof.

EP 0 363 083 A2

# Fig. 1

Graph with X-axis: TIME AFTER INJECTION (HOURS), values 0, 6, 12, 18.
Left Y-axis: NUMBER OF PLATELETS ($\times 10^4 / \mu\ell$), values 100, 150, 200.
Right Y-axis: AChE$^+$CELLS/ NUCLEATED CELLS (%), values 0.05, 0.10, 0.15.

Legend:

—○— CULTURE SUPERNATANT
(NUMBER OF PLATELETS)

—△— CULTURE SUPERNATANT
(RATIO OF AChE POSITIVE CELLS)

—●— MEDIUM (NUMBER OF PLATELETS)

—▲— MEDIUM (RATIO OF AChE POSITIVE CELLS)

# Fig. 2

Figure 2: Graph showing NUMBER OF PLATELETS ($\times 10^4/\mu\ell$) and AChE$^+$CELLS / NUCLEATED CELLS (%) versus DAYS AFTER START OF INJECTION.

Legend:

—◯— CULTURE SUPERNATANT (NUMBER OF PLATELET)

—△— CULTURE SUPERNATANT (RATIO OF AChE POSITIVE CELLS)

—●— MEDIUM (NUMBER OF PLATELETS)

—▲— MEDIUM (RATIO OF AChE POSITIVE CELLS)

# Fig. 3

NUMBER OF PLATELETS $(\times 10^4/\mu\ell)$

MEDIUM EXCHANGE

CULTURE DAY

●———● NUMBER OF PLATELETS IN MOUSE INJECTED WITH SUPERNATANT FROM MEDIUM CONTAINING IL-1α, AND CULTURING PERIOD IN THIS CONDITION

○---○ NUMBER OF PLATELETS IN MOUSE INJECTED WITH SUPERNATANT FROM MEDIUM NOT CONTAINING IL-1α, AND CULTURE IN THIS CONDITION

*** $P < 0.001$     ** $P < 0.01$

* $P < 0.05$

# Fig. 4

INJECTION

NUMBER OF PLATELETS (×10⁴/μℓ)

DAYS AFTER START OF INJECTION

−○− MEDIUM

−●− MEDIUM CONTAINING IL-1α

−△− CULTURE SUPERNATANT FROM MEDIUM CONTAINING IL-1α

∗∗∗ P < 0.001          ∗∗ P < 0.01

∗ P < 0.05

# Fig. 5

—O— MEDIUM

—△— CULTURE SUPERNATANT FROM
MEDIUM CONTAINING IL-1$\alpha$

$*$ P < 0.001

# *Fig. 6*

① BOVINE SERUM ALBUMIN (67,000)
② OVALBUMIN (43,000)
③ β-LACTOGLOBULIN (36,800)
④ CHYMOTRYPSINOGEN (25,000)
⑤ CYTOCROME C (12,300)

## Fig. 7

DEAE-SEPHAROSE CL-6B ION-EXCHANGE CHROMATOGRAM

ⓘ 20mM Tris-HCℓ BUFFER(pH7.5) 100mM NaCℓ

ⓘⓘ 20mM Tris-HCℓ BUFFER(pH7.5) 200mM NaCℓ

ⓘⓘⓘ 20mM Tris-HCℓ BUFFER(pH7.5) 1000mM NaCℓ

EP 0 363 083 A2

# Fig. 8

# Fig. 9

EP 0 363 083 A2

*Fig. 10*

EP 0 363 083 A2

*Fig. 11*

1 2

92.5 kd →
66.2 kd →

45 kd →

31 kd →

21.5 kd →

14.4 kd →

1 MOLECULAR WEIGHT STANDARDS
2 PURIFIED TPO

*Fig. 12*

92.5

66.2

45

31 ── 27 K
── 24 K

21.5

14.4

1 2

1 MOLECULAR WEIGHT STANDARDS
2 PURIFIED TPO

# Fig. 13

o ··· 2% FCS/DMEM
● ··· 0.2μg TPO
△ ··· 2μg TPO
** ··· P< 0.01
*** ··· P< 0.001

Fig. 14

# Fig. 15

**X-axis:** BSF₂ CONCENTRATION (U/mℓ)

**Y-axis:** NUMBER OF MEGAKARYOCYTES (×10⁵)